# DEMANDE DE BREVET EUROPEEN

(11) **EP 0 626 446 A1**
(43) Date de publication de la demande: **30.11.1994**
(21) Numéro de dépôt: 94400970.3
(22) Date de dépôt: 04.05.1994
(51) Int. Cl.: C12N 1/26, C12N 1/00

(54) **Milieu de culture d'une flore microbienne procédé de préparation d'un inoculum lyophilise et inoculum obtenu par ce procédé**

(30) Priorité: 06.05.1993 FR 9305436
(71) Demandeur: Société Anonyme dite : ELF ANTAR FRANCE, F-92400 Courbevoie (FR)
(72) Inventeur: Gagneux, Joelle, F-69009 Lyon (FR)
(74) Mandataire: Des Termes, Monique

(57) **Abrégé**

Le domaine de l'invention est celui de la biologie appliquée à la mise au point d'additifs ayant des propriétés biocides, destinés à protéger des milieux dans lesquels des microorganismes se développent naturellement.

L'invention a pour objet la préparation d'un milieu de culture d'une flore microbienne, un procédé de préparation d'un inoculum lyophilisé, et l'inoculum lyophilisé lui-même. Selon l'invention le milieu de culture a la particularité de permettre le développement simultané de bactéries, de levures et de champignons.

L'invention trouve une application privilégiée dans la réalisation de tests de résistance des huiles pour travail des métaux, aux attaques des microorganismes.

## Description

### DOMAINE TECHNIOUE

L'invention concerne un milieu de culture d'une flore microbienne, un procédé de préparation d'un inoculum lyophilisé, et l'inoculum obtenu par ce procédé. Elle trouve son application générale dans le domaine de la biologie appliquée à l'étude du développement simultané de microorganismes dans divers milieux pour la préparation d'inocula destinés à des travaux de laboratoire ainsi que dans l'industrie pour effectuer des tests comparatifs de développement de microorganismes.

Elle trouve une application privilégiée dans la réalisation de tests de résistance des huiles pour travail des métaux, aux attaques de microorganismes qui s'y développent naturellement. Ces huiles pour travail des métaux sont obtenues par mélange de lubrifiant d'origine pétrolière ou synthétique avec de l'eau et sont largement utilisées dans les ateliers d'usinage des métaux au moyen de machines outils.

L'invention n'est nullement limitée à cette application type, elle est aussi applicable à l'étude du développement simultané de microorganismes dans d'autres milieux tels que les sols, les substrats de cultures artificielles, les eaux résiduaires des raffineries de pétrole brut, divers milieux des industries Agro-alimentaires.

### ETAT DE LA TECHNIOUE ANTERIEURE

Pour effectuer des études de développement simultané de microorganismes, dans divers fluides ou solides, et en particulier pour réaliser des tests comparatifs de l'efficacité de différents biocides destinés à protéger des fluides ou des solides dans lesquels des microorganismes se développent concurremment et naturellement, il est nécessaire de disposer d'une flore microbienne représentative de ces microorganismes et d'un milieu de culture permettant la croissance de cette flore microbienne.

Les milieux de culture de flores microbiennes sont nombreux et permettent en particulier le développement séparé de bactéries, de champignons et de levures, mais comme la prolifération des bactéries inhibe la croissance des champignons et des levures, qui ont une cinétique de croissance beaucoup plus lente, aucun des milieux connus ne permet le développement simultané des trois espèces, bactéries, champignons et levures, qui constituent la flore microbienne qui colonise par exemple les huiles pour travail des métaux.

### EXPOSE DE L'INVENTION

La présente invention a justement pour objet de remédier à cet inconvénient, et notamment de fournir un milieu de culture d'une flore microbienne, un procédé de préparation d'un inoculum lyophilisé, et l'inoculum obtenu par ce procédé, contenant des bactéries, des levures et des champignons capables de se développer concurremment.

A cette fin la présente invention propose un milieu de culture permettant le développement simultané de bactéries, de levures et de champignons, caractérisé en ce qu'il comprend de l'eau, du bouillon de malt, du bouillon nutritif, de l'eau peptonée, du milieu Sabouraud liquide et au moins un lubrifiant.

On précise que le bouillon de malt est une solution aqueuse d'extrait de malt, obtenue à partir d'orge germée moulue. Généralement, il contient 10g/l de malt.

Le bouillon nutrifif est une solution aqueuse de viande. Il comprend généralement 500ml de macération de viande et 250ml de digestion pepsique de viande pour 250ml d'eau, pH final de 7,4.

L'eau peptonée peut être, soit une solution de digestion pepsique de viande et d'hydrolyse d'estomac de porc, soit une solution de peptone pancréatique d'organes type ST.

A titre d'exemple, la première formule d'eau peptonée peut comprendre pour 1l, 500ml de digestion pepsique de viande et 500ml d'hydrolyse d'estomac de porc ; la deuxième formule d'eau peptonée peut comprendre pour 1l d'eau, 20g de peptone pancréatique d'organes type ST et 5g de chlorure de sodium, pH final 7,2-7,3.

Le milieu Sabouraud liquide est une solution de peptone et de glucose ou maltose massé. Il peut contenir par exemple 10g de peptone telle que la peptone pepsique de viande de boeuf et 20g de glucose ou de maltose pour un litre d'eau.

Les différents constituants du milieu de culture de l'invention sont utilisés sous la forme de poudres, telles qu'elles sont commercialisées par les fabricants. Aussi, on prépare le milieu de culture de l'invention en ajoutant ces différentes poudres à de l'eau.

Selon une autre caractéristique du milieu de culture de l'invention, le lubrifiant est constitué par un lubrifiant non protégé par des additifs ayant des propriétés biocides, convenant à la préparation d'une huile pour travail des métaux.

Selon une autre caractéristique de la présente invention, le milieu de culture comprend pour un litre d'eau:
- de 3 à 5g de poudre pour bouillon de malt,
- de 2 à 4g de poudre pour bouillon nutritif,
- de 1 à 3g de poudre pour eau peptonée,
- de 2 à 4g de poudre pour milieu Sabouraud liquide, et
- de 9 à 11ml de lubrifiant.

Ce milieu de culture convient en particulier pour le développement simultané des bactéries, des champignons et des levures qui se trouvent habituellement dans les huiles pour travail des métaux. A titre d'exemple les bactéries peuvent être Pseudomonas oléovorans, Pseudomonas aéruginosa, Citrobacter freundii, Escherichia coli, Proteus vulgaris, les levures sont de type Candida Sp et les champignons peuvent être de type Fusarium solani.

L'invention a également pour objet un procédé de préparation d'un inoculum liquide, caractérisé en ce qu'il consiste à ensemencer un milieu de culture par une flore microbienne comprenant des bactéries, des levures et des champignons susceptibles de se développer dans des huiles pour travail des métaux et à cultiver cette flore microbienne dans ledit milieu dans des conditions aérobies.

Selon ce procédé on réalise de préférence la culture sous aération par léger bullage d'air comprimé, à une température de 23 à 26°C, pendant une durée de 7 à 9 jours environ.

La flore microbienne utilisée pour ensemencer le milieu de culture peut être obtenue en mélangeant des souches de bactéries, de levures et de champignons cultivées séparément et provenant d'un mélange d'huiles pour travail des métaux très contaminées.

Bien entendu, on peut également y ajouter certaines souches pures provenant d'une collection de microorganismes.

On peut aussi préparer la flore microbienne destinée à ensemencer le milieu de culture en mélangeant des souches pures de collection de bactéries, de levures et de champignons représentatives de la flore microbienne souhaitée, obtenues par culture séparée.

La présente invention a encore pour objet un procédé de préparation d'un inoculum lyophilisé, mixte et stable, caractérisé en ce que l'on prépare un inoculum liquide par le procédé décrit ci-dessus puis on mélange cet inoculum liquide avec du lait stérilisé demi-écrémé, dépourvu d'antibiotique, et on soumet ledit mélange à une lyophilisation.

L'invention a aussi pour objet l'inoculum lyophilisé conditionné en flacons obtenu par le procédé décrit ci-dessus, caractérisé en ce qu'il contient au moins 10⁹ bactéries par flacon de 10ml, des levures et des champignons capables de se développer simultanément dans différents milieux, notamment des huiles pour travail des métaux.

### EXPOSE DETAILLE DE L'INVENTION

D'une manière générale, le milieu de culture et le procédé de l'invention sont utilisés pour préparer un inoculum lyophilisé contenant des bactéries, des levures et des champignons, capables de se développer notamment dans des huiles pour travail des métaux.

L'invention a consisté à mettre au point un milieu contenant des éléments capables d'assurer le développement de toute la flore qui se développe naturellement dans les huiles pour travail des métaux, ce milieu contenant de l'eau, du bouillon de malt, du bouillon nutritif, de l'eau peptonée, du milieu Sabouraud liquide et au moins un lubrifiant utilisé pour préparer de l'huile pour travail des métaux, ce lubrifiant étant exempt de biocides pour ne pas gêner le développement de la flore microbienne.

Les concentrations des différents constituants ont été recherchées expérimentalement pour que les microorganismes se développent correctement et que l'on puisse obtenir en fin d'opération une poudre par lyophilisation du milieu de culture. Le milieu qui satisfait à ces conditions comprend pour un litre d'eau :
- de 3 à 5g de poudre pour bouillon de malt,
- de 2 à 4g de poudre pour bouillon nutritif,
- de 1 à 3g de poudre pour eau peptonée,
- de 2 à 4g de poudre pour milieu Sabouraud liquide, et
- de 9 à 11ml de lubrifiant.

Le milieu ainsi préparé est ensemencé par une flore de même nature que celle de l'inoculum que l'on veut préparer, constituée de bactéries par exemple : Pseudomonas oléovorans, Pseudomonas aéruginosa, Citrobacter freundii, Escherichia coli, Proteus vulgaris, et/ou des levures Candida Sp et des champignons Fusarium solani.

Le milieu étant ensemencé, la culture de la flore microbienne se réalise dans des conditions aérobies, obtenues par exemple par aération du milieu au moyen d'un léger bullage à l'air comprimé. La culture est réalisée à une température de 23 à 26°C particulièrement adaptée à la flore pendant une durée de 7 à 9 jours.

Selon un premier mode de préparation, la flore microbienne destinée à ensemencer le milieu de culture est obtenue en mélangeant des souches de bactéries cultivées séparément.

Pour obtenir ces souches, on peut partir des huiles pour travail des métaux, d'origines variées, très contaminées naturellement, et procéder à des étalements sur P.C.A (Plate Count Agar), sur gélose Sabouraud et sur gélose de malt selon des méthodes bien connues. Ensuite on récupère toutes les populations qui se sont développées séparément et on les mélange pour ensemencer le milieu de culture.

Selon un deuxième mode de préparation, la flore microbienne destinée à ensemencer le milieu de culture est obtenue en mélangeant des souches pures de bactéries, de champignons et de levures provenant de collections de cultures de microorganismes cultivés en laboratoire selon des méthodes connues, de même nature que ceux susceptibles de se développer naturellement dans les huiles pour travail des métaux ou dans d'autres milieux par exemple, le sol et des eaux résiduaires de diverses industries.

L'inoculum liquide obtenu selon l'une des deux méthodes de préparation est ensuite mélangé à du lait demi-écrémé stérilisé, dépourvu d'antibiotique, qui sert de substrat de protection des microorganismes, en particulier des bactéries, pendant la phase de congélation qui sera décrite avec le procédé de lyophilisation.

Pour que le produit préparé présente les caractéristiques souhaitées, l'inoculum liquide doit être mélangé à un volume sensiblement équivalent de lait demi-écrémé, le mélange obtenu étant lyophilisé selon le procédé suivant:

Le mélange inoculum liquide/lait est introduit à raison de 10ml dans des flacons de 25 à 35ml. L'ensemble des flacons est alors porté à une température de -40°C et maintenu à cette température pendant une durée de 10 à 12 heures.

Les congelats ainsi obtenus sont ensuite soumis à une dessication sous vide. Le produit résultant est une poudre blanche compacte qui peut être conservée sans altération pendant au moins une année. Lors de l'utilisation, on reconstitue l'inoculum liquide en mettant en solution dans 10ml d'eau la poudre contenue dans chaque flacon. On obtient ainsi un inoculum liquide qui contient au moins 10⁹ bactéries/ml, des levures et des champignons capables de se développer simultanément dans différents milieux, notamment des huiles pour travail des métaux.

### EXEMPLE DE PREPARATION D'INOCULUM

Généralement les lubrifiants utilisés pour préparer les huiles de travail des métaux sont constitués principalement d'hydrocarbures, auxquels on ajoute des additifs, on peut citer des anticorrosifs, des tensioactifs, des émulgateurs anioniques, non ioniques, des agents antiusure, des additifs d'onctuosité et extrême pression. Ces lubrifiants sont utilisés sous forme de solutions ou d'émulsions pour concilier les propriétés de lubrification et de pouvoir refroidissant nécessaires à la durée de vie des outils d'usinage. Ces huiles de travail des métaux sont généralement obtenues par dilution de 2 à 10% de lubrifiant additivé dans de l'eau

A titre d'exemple, on décrit ci-après la préparation d'un inoculum lyophilisé utilisant la flore microbienne provenant d'un mélange d'huiles usagées de travail des métaux ayant été prélevées dans trois usines de fabrication de pièces d'automobiles.

Les bactéries, champignons et levures destinés à ensemencer le milieu de culture sont cultivés séparément de la manière suivante :

On procède à des étalements sur P.C.A., sur gélose Sabouraud et sur gélose de malt en étalant une goutte de mélange des huiles pour travail des métaux usagés sur chacun de ces milieux disposés dans des boîtes de Pétri. La boîte de Pétri qui contient le milieu P.C.A. est maintenue à une température de 35°C pendant 24 heures pour développer les bactéries, celle qui contient la gélose Sabouraud est maintenue à 20°C pendant 3 jours pour développer les levures et celle qui contient la gélose de malt est maintenue à 20°C pendant 5 jours pour développer les champignons.

Ces opérations de cultures séparées étant terminées, on récupère la totalité des microorganismes qui se sont développés, on les mélange à un litre du milieu de culture qui contient pour un litre d'eau :
- 4g de poudre pour bouillon de malt
- 3g de poudre pour bouillon nutritif
- 2g de poudre pour eau peptonée
- 10ml de lubrifiant non protégé.

Le milieu étant ainsi préparé et ensemencé, la culture de la flore microbienne se réalise dans les conditions aérobies obtenues par aération, par bullage à l'air comprimé, à une température de 25°C pendant 10 jours.

L'inoculum ainsi obtenu contient 10⁸ bactéries/ml, des champignons et des levures visibles au microscope.

Un échantillon de cet inoculum est analysé par un laboratoire de microbiologie qui dénombre les bactéries présentes et vérifie que cet échantillon est exempt de souches à caractère potentiellement pathogène.

L'inoculum liquide est ensuite mélangé à un volume équivalent de lait demi-écrémé stérilisé. Le mélange obtenu est introduit à raison de 10ml dans des flacons de 30ml. Ensuite on procède à la lyophilisation de la manière suivante :

L'ensemble des flacons est porté à une température de -40°C et maintenu à cette température pendant une durée de 12 heures. Les congelats ainsi obtenus sont soumis à une dessication sous vide. Le résultat de cette opération est une poudre blanche compacte.

Lors de l'utilisation on reconstitue un inoculum liquide en ajoutant au contenu de chaque flacon 10ml d'eau distillée.

On vérifie ensuite en laboratoire que cet inoculum contient plus de 10⁸ bactéries par ml, ainsi que des champignons et des levures.

## Revendications

**1-** Milieu de culture permettant le développement simultané de bactéries, de levures et de champignons, caractérisé en ce qu'il comprend de l'eau, du bouillon de malt, du bouillon nutritif, de l'eau peptonée, du milieu Sabouraud liquide et au moins un lubrifiant.

**2-** Milieu de culture selon la revendication 1, caractérisé en ce que le lubrifiant est un lubrifiant non protégé par des additifs ayant des propriétés biocides convenant à la préparation d'une huile pour travail des métaux.

**3-** Milieu de culture selon l'une quelconque des revendications 1 et 2, caractérisé en ce qu'il comprend pour un litre d'eau :
- de 3 à 5g de poudre pour bouillon de malt,
- de 2 à 4g de poudre pour bouillon nutritif,
- de 1 à 3g de poudre pour eau peptonée,
- de 2 à 4g de poudre pour milieu Sabouraud liquide, et
- de 9 à 11ml de lubrifiant.

**4-** Milieu de culture selon l'une quelconque des revendications 1 à 3, caractérisé en ce que les bactéries sont choisies parmi Pseudomonas oléovorans, Pseudomonas aéruginosa, Citrobacter freundii, Escherichia coli, et Proteus vulgaris, les levures sont du type Candida Sp et les champignons sont du type Fusarium solani.

**5-** Procédé de préparation d'un inoculum liquide, caractérisé en ce qu'il consiste à ensemencer un milieu de culture selon l'une quelconque des revendications 1 à 4 par une flore microbienne comprenant des bactéries, des levures et des champignons susceptibles de se développer dans des huiles pour travail des métaux et à cultiver cette flore microbienne dans ledit milieu dans des conditions aérobies.

**6-** Procédé selon la revendication 5, caractérisé en ce que l'on réalise la culture sous aération par léger bullage d'air comprimé, à une température de 23 à 26°C pendant une durée de 7 à 9 jours environ.

**7-** Procédé selon l'une quelconque des revendications 5 et 6, caractérisé en ce que l'on prépare la flore microbienne destinée à ensemencer le milieu de culture en mélangeant des souches de bactéries, de levures et de champignons cultivés séparément en provenance d'un mélange d'huiles pour travail des métaux très contaminées.

**8-** Procédé selon l'une quelconque des revendications 5 et 6, caractérisé en ce que l'on prépare la flore microbienne destinée à ensemencer le milieu de culture en mélangeant des souches pures de collection de bactéries, de levures et de champignons représentatives de la flore microbienne souhaitée, obtenues par cultures séparées.

**9-** Procédé selon l'une quelconque des revendications 5 et 6, caractérisé en ce que l'on prépare la flore microbienne destinée à ensemencer le milieu de culture en mélangeant des souches de bactéries, de levures et de champignons cultivés séparément en provenance d'un mélange d'huiles pour travail des métaux très contaminées, et en y ajoutant des souches pures de collection représentatives de la flore microbienne souhaitée, obtenues par cultures séparées.

**10-** Procédé de préparation d'un inoculum lyophilisé, mixte et stable, caractérisé en ce que l'on prépare un inoculum liquide par le procédé selon l'une quelconque des revendications 5 à 9, on mélange cet inoculum liquide avec du lait stérilisé demi-écrèmé, dépourvu d'antibiotique, et on soumet ledit mélange à une lyophilisation.

**11-** Procédé selon la revendication 10, caractérisé en ce que le rapport en volume de l'inoculum au lait est sensiblement égal à 1.

**12-** Procédé selon la revendication 10, caractérisé en ce que, pour réaliser la lyophilisation, on introduit le mélange dans des flacons de 25 à 35ml à raison de 10ml de mélange par flacon, on porte l'ensemble des flacons à une température de -40°C pendant 10 à 12h pour obtenir des congelats qui sont ensuite soumis à une dessication.

**13-** Inoculum lyophilisé conditionné en flacons obtenu par le procédé selon l'une quelconque des revendications 10 à 12, caractérisé en ce qu'il contient au moins 10⁹ bactéries par flacon, des levures et des champignons capables de se développer simultanément dans différents milieux, notamment des huiles pour travail des métaux.
